# EUROPEAN PATENT APPLICATION

(11) **EP 2 676 665 A1**
(43) Date of publication of application: **25.12.2013**
(21) Application number: 12746907.0
(22) Date of filing: 14.02.2012
(51) Int. Cl.: A61K 31/352, A61K 9/20, A61K 9/14, A61K 9/48, A61K 9/19, A61P 25/28

(54) **USE OF -MANGOSTIN IN PREPARATION OF MEDICAMENTS FOR TREATING ALZHEIMER'S DISEASE**

(30) Priority: 18.02.2011 CN 201110040746
(71) Applicant: Bomai Limited, Hangzhou, Zhejiang (CN)
(72) Inventor: XIA, Zheng, Hangzhou, Zhejiang 310016 (CN)
(74) Representative: Edlund, Fabian
(86) International application number: PCT/CN2012/071112
(87) International publication number: WO 2012/109976

(57) **Abstract**

The invention discloses a new use of α-Mangostin in medicine, which is application of α-Mangostin in preparation of medicaments for Alzheimer's disease. Under the new working concentration, α-Mangostin showed the capability to inhibit the aggregation and deposition of Aβ. Moreover, it displayed the neuroprotective effect against neurotoxicity caused by Aβ oligomer, which maintained normal morphology of mammalian neuron cells, enhanced their normal physiological function, and realized the intervention in pathological process of Alzheimer's disease. The invention provided a new approach to treat Alzheimer's disease.

## Description

### Technical Field

This invention relates to the fields of pharmacology and chemical biology, particularly application of α-Mangostin in preparation of medicaments for Alzheimer's disease.

### Background of the Invention

Alzheimer's disease is a progressive fatal neurodegenerative disease manifesting cognitive and memory deterioration, proceeding diminishing of daily activities, with a variety of neurological symptoms and behavioral disorders.

Aβ aggregation and deposition is an important pathological process in the development of Alzheimer's disease. During the process of Aβ aggregation and deposition, Aβ oligomer with strong nerve toxicity, and senile plaque, one of the pathological hallmarks of Alzheimer's disease, can be formed. Studies have shown that injecting the aggregation of Aβ can induce the occurrence of Alzheimer's disease-like symptoms in mice, while the pathological symptoms of Alzheimer's disease could be relieved by inhibiting Aβ aggregation and deposition, such as the capability of learning and memorializing could be improved in both preclinical (cell models or animal models) and clinical researches.. Therefore, the inhibitors of Aβ aggregation and deposition have been promised the potential application in the treatment of Alzheimer's disease, and the development of such inhibitors has become an important direction in the studies of Alzheimer's disease.

α-Mangostin is an extract from garcinia mangostana (also known as Garcinia mangostana L., garcinia mangostana, or mangosteen) used as a traditional medicine in Southeast Asia, which mainly consists in a nutshell of mangosteen and now can be synthesized artificially. Structure formula of α-Mangostin is as follows:

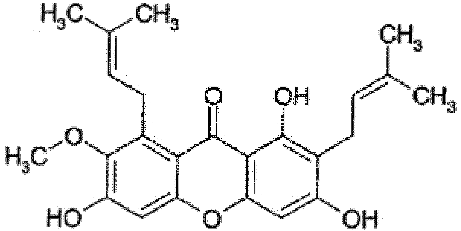

It has been reported that under certain working concentration, α-Mangostin can produce the inhibition on acid sphingomyelinase, topoisomerase I and topoisomerase II. Meanwhile α-Mangostin is a competitive antagonist of Histamine H1-receptor, which could be applied in clinical to treat anaphylaxis caused by histamine release.

Up to present, there is no report of applying α-Mangostin on inhibiting Aβ aggregation and deposition and preparing medicaments for Alzheimer's disease.

### Summary of the Invention

The object of the invention is to overcome defects and deficiencies of the existing technology, providing a new use of α-Mangostin in medicine, which is the application of α-Mangostin in preparation of medicaments for Alzheimer's disease.

α-Mangostin described in the invention can be natural extracts or synthetic chemicals. By incubating α-Mangostin and Aβ together, it is proven that α-Mangostin obviously inhibits Aβ aggregation and deposition. Adding α-Mangostin before or after the formation of Aβ oligomer, α-Mangostin could decrease the content of Aβ oligomer. When applying α-Mangostin in mammalian cells, it is found that α-Mangostin has neuroprotective effect against neurotoxicity caused by Aβ oligomer, which maintains normal morphology of mammalian neuron cells and enhances their normal physiological function. Experimental results show that there is corresponding improvements in cellular functions, including membrane permeability, mitochondrial transmembrane potential, and karyomorphism etc. When applying α-mangostin to mammals with Alzheimer's disease by suitable pharmaceutical preparation, it is able to significantly improve learning and memorizing capability of mammals, and thereby produce the effects of treating Alzheimer's disease.

In a preferred embodiment, a medication involving α-mangostin is administered orally. Dosage range of α-mangostin for people is recommended from 50ng/kg to 200µg/kg. The preferred dosage range is recommended from 500ng/kg to 50µg/kg. Referencing disclosure of the invention, the technicians will understand that dosage range for people in any preparation refers to the following formula: dosage for people ≈ dosage for mice/12. Lower than the working concentration of as a Histamine H1-receptor antagonist (generally higher than 10⁻⁶ mol/L), α-mangostin shows the effect to inhibit Aβ aggregation and deposition, which realizes the intervention in pathological process and improvement in pathological symptoms of Alzheimer's disease.

A medication involving α-mangostin in the invention can be administered by injection, including hypodermic injection and intravenous injection.

A medication involving α-mangostin in the invention can be tablet, granule, medicinal capsule, powder-injection, and sustained or controlled release preparations.

Under the working concentration, α-mangostin shows the effect to inhibit Aβ aggregation and deposition. Meanwhile, α-mangostin has neuroprotective effect against neurotoxicity caused by Aβ oligomer, enhancing normal physiological function of mammalian neurons cells, maintaining normal morphology of mammalian neurons, and realizing the intervention in pathological process of Alzheimer's disease. The invention provides a new approach to treat Alzheimer's disease.

In the invention, there are some terms to describe Alzheimer's disease, such as Aβ, Aβ aggregation and deposition, Aβ oligomer, neuroprotective effect, neurotoxicity, improving learning and memorizing capability (escape latency and distance), absorbance value, linear relation, positive correlation, statistical significance, etc. As all above terms are applied commonly in science as ordinary scientific terminology, they will not in any way limit the scope of the invention.

### Brief Description of the Drawings

With reference to the figures, the detailed embodiments of the present invention are described.

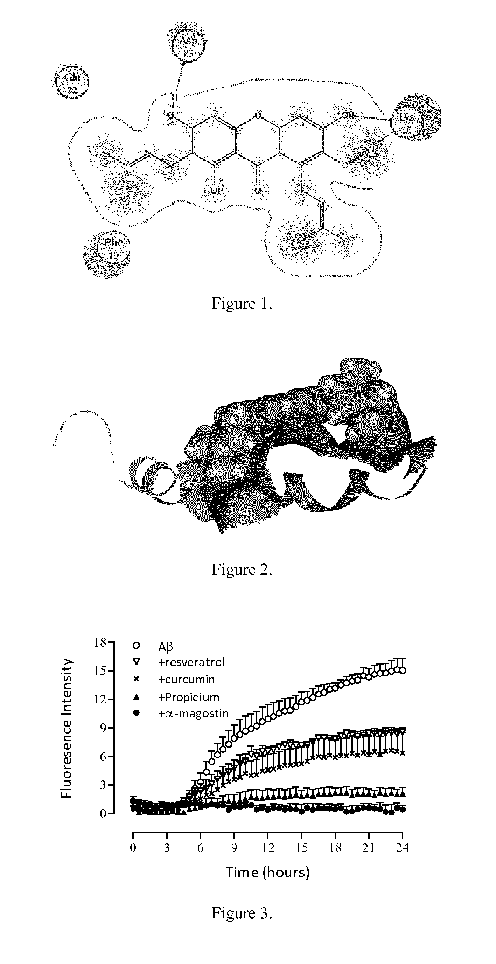

Figure 1 illustrates the docking model in which α-Mangostin binds to Aβ. Therein, phenolic hydroxyl groups at positions 3 and 6, 7 of α-Mangostin bind to Asp23 and Lys16 of Aβ by hydrogen bonds, respectively. In addition, α-Mangostin also has a direct interaction with Phe19 and Glu22 of Aβ, displaying mainly as the force of π-π conjugation between benzene rings and Van der Waals force.

Figure 2 shows the maintained α helical conformation of Aβ when binding with α-Mangostin.

Figure 3 demonstrates the inhibiting experimental results of various inhibitors act on Aβ aggregation. Therein, Aβ aggregation is shown by fluorochrome of Thioflavin-T. The stronger fluorescence intensity it shows, the more serious Aβ aggregation it happens. Resveratrol, Curcumin and Propidium Iodide are the reported inhibitors on Aβ aggregation. When these inhibitors and α-Mangostin applied on Aβ (with molar ratio of inhibitor: Aβ as 1:1) and incubated at 37°C, α-Mangostin shows the greatest capability to inhibit Aβ aggregation wherein Aβ aggregation is almost completely inhibited within 24 hours.

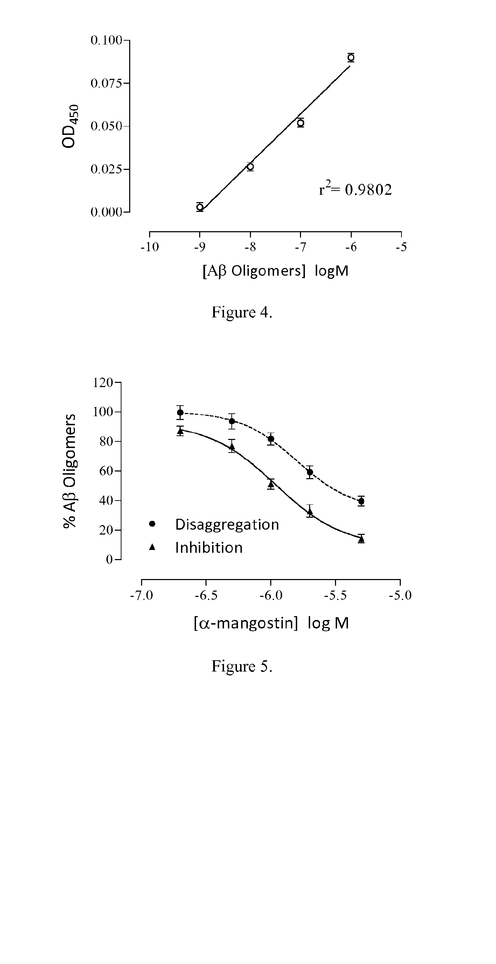

Figure 4 shows the linear relation between Aβ oligomer concentration and its optical density. Therein, Aβ oligomer concentration is measured by ELISA assay. In a certain range of concentration, Aβ oligomer concentration is linear with its optical density (OD450), the square of the coefficient of determination is 0.98 (R²=0.98).

Figure 5 is the curve graph that illustrates Aβ oligomer is disaggregated by α-Mangostin. When α-Mangostin is co-incubated with Aβ monomers, the formation of Aβ oligomer is significantly inhibited, which shows a positive correlation with the mass of α-Mangostin that was added. When α-Mangostin is applied into the formed Aβ oligomers, Aβ oligomers are disaggregated to monomers, also in an α-Mangostin concentration dependent manner.

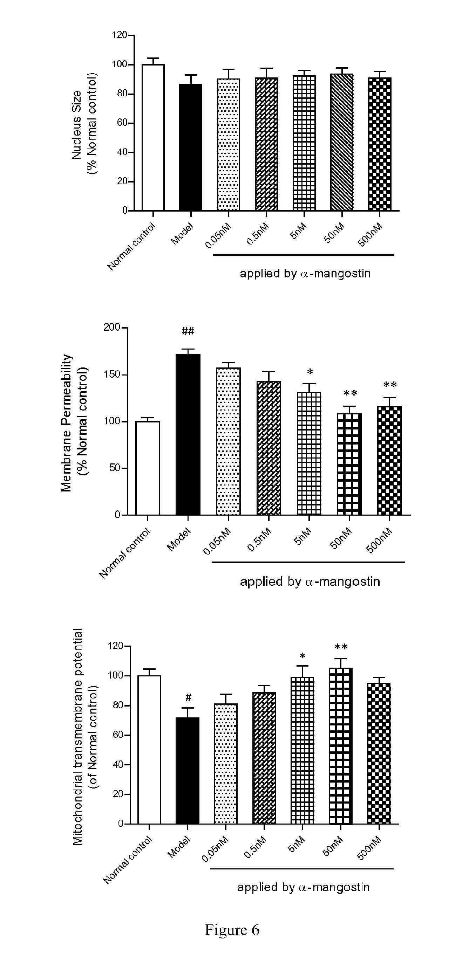

Figure 6 demonstrates the neuroprotective experimental results of α-Mangostin. Therein, Aβ oligomer's neurotoxicity performs as its effect on nucleus size, membrane permeability and mitochondrial transmembrane potential. α-Mangostin can recover those properties of neurons with a bell-shaped concentration-effect curve. # indicates P < 0.05, compared between the model group and control. * indicates P < 0.05 and ** indicates P < 0.01, compared between α-Mangostin treatment group and the model group.

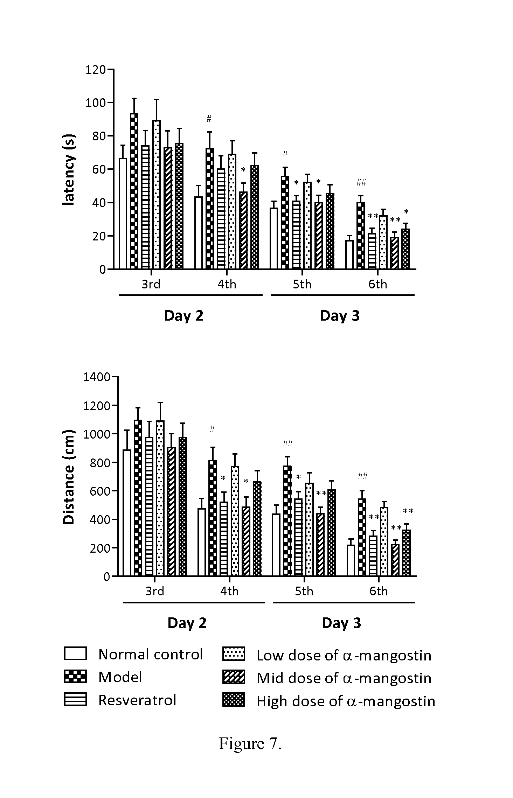

Figure 7 demonstrates experimental results that α-Mangostin improves the cognitive function in the animal model of Alzheimer's disease (SAM-P8 mice). Therein, SAM-P8 mice are widely accepted as the animal model for Alzheimer's disease in pharmacodynamics study. It appears obvious symptoms of Alzheimer's disease after being fed for a certain period (older than six months age), and its escape latency time and swim distance are both longer than that of normal mice in water maze test. These cognitive symptoms can be significantly relieved by α-Mangostin # indicates P < 0.05, compared between the model group and control. * indicates P < 0.05 and ** indicates P < 0.01, compared between α-Mangostin treatment group and the model group.

### Detailed Description of The Embodiments

The embodiments are provided as follow, which discloses the methods of how to prepare, use and evaluate the medications in the invention, but does not limit the scope and content of the invention. Although the data herein (such as quantity, concentration, and others) are tried to be correct as far as possible in the embodiments, some certain experimental errors and variations are still allowed.

Material and methods.

Female SAM-P8 mice of 8-month old were employed as the model animal in the invention. All of the mice experimental procedures strictly followed the Guide for the Care and Use of Laboratory Animals as adopted and promulgated by the National Institutes of Health, USA, including sterile rearing environment, temperature controlled between 23°C and 25°C, humidity controlled at 55±5%, and an interval of twelve hours light.

Mammalian neurons in the experiment were prepared from embryonic day 15 SD rat embryos. A medium specific for neurons was employed, including neurobasal, 2% B27 and 1% glutamine that were all commercial and used following the instructions of the manufacturer.

Source of α-Mangostin in the invention was the plant extract or chemical synthesis by the GMP manufacture. Aβ was bought from Sigma-Aldrich Corporation (China) with HPLC purity higher than 98%. Other materials in the invention were also commercial and used following the instructions of the manufacturer.

The concentration and dose of α-Mangostin applied in the experiment were adjusted according to the different animal models. In molecular experiments, α-Mangostin was employed to calibrate the concentration of Aβ, prepared with the unit of molar concentration, and compared with the known Aβ aggregation inhibitor. In cellular experiments, α-Mangostin was also prepared with the unit of molar concentration, and provided in screening from the concentration of 50 pmol/L to 500nmol/L. In animal experiments, following the established practice, α-Mangostin was prepared with the unit of weight/ animal weight, provided dosage range across three orders of magnitude of low dose (1µg/kg), mid dose (10µg/kg), high dose (100µg/kg), and administrated orally (O.P., Solid granules).

Embodiment 1, the mode of α-Mangostin binding on Aβ is simulated by computer.

NMR structure of Aβ₁₋₄₀ (PDB: 1BA4) was loaded in molecular simulation software MOE. Three-dimensional structure of Aβ₁₋₄₀was built with the program of Partial Charges and Energy Minimize of MOE after hydrones and other molecules added in NMR experiment were removed and hydrogens lost in NMR experiment were filled.

Two-dimensional structure of α-Mangostin was built in molecular simulation software MOE, and the three-dimensional structure was built with the program of Partial Charges and Energy Minimize of MOE.

3D α-Mangostin structure was docked into the 3D structure of Aβ₁₋₄₀ that has been removed the flexible region (1-13), utilizing the automatic docking program of MOE, and the top 20 binding modes were chose to obtain the most optimized mode by charges equilibration and energy minimization.

As shown in Fig. 1, α-Mangostin bound on Aβ from residue 16 to 23. This region, full of polar amino acids, was the β-turn region that plays a key role in Aβ conformational change from α-helix to β hairpin. Phenolic hydroxyl group at position 3 and 6, 7 of α-Mangostin bound to Asp23 and Lys16 of Aβ by hydrogen bond, respectively. The benzene ring in xanthene of α-Mangostin formed a π-π conjugation with Phe19 of Aβ. By these bindings, α-Mangostin fit within the key region of Aβ conformational change, which stabilized α-helical conformation or change β hairpin conformation back to α-helix (as shown in Fig. 2) . The structure of Aβ bond by α-Mangostin was almost the same as that of Aβ α-helix itself (RSMD =0.91A°), and the free energy of α-Mangostin binding to Aβ was -68.76 KCal/mol.

In embodiment 2, fluorescence kinetics was applied to measure α-Mangostin inhibition on Aβ aggregation.

1mg Aβ was dissolved in 500µL HFIP, and put at room temperature for 120 minutes with intermittent shaking. Then HFIP was dried by high pure nitrogen blowing and 100µlL DMSO was added to prepare an Aβ stock of 2.3mM, which was stored at -20°C. DMSO was employed to dilute Aβ stock and 2µL diluted solution was added to 16µL PBS (0.215M, pH 8.0), which made a solution of Aβ at 25µM, to which 2µL α-Mangostin (25µM) or other inhibitors, and blank solvent was added. Followed with incubation for 30 minutes, the mixture was added 80µL Thioflavin-T(10µM)/Glycine-NaOH (50mM, pH8.5) and transferred to microplate for fluorescence intensity measurement. The parameters for measurement was set up as follow: instrument temperature of 37°C, shaking frequency of 240rpm, radius of 2nm, excitation wavelength of 446nm, emission wavelength of 485nm, bandwidth of 5nm, and detection frequent of every 30 minute. Fluorescence intensity was recorded and statistically analyzed.

As shown in Fig. 3, the fluorescence kinetics curve demonstrates three phases of Aβ incubation as latency, accumulation and plateau. Therein, Aβ incubated itself entered into accumulation after incubation for 4 hours (take-off point), which was embodied in the notable increase of fluorescence and the absence of plateau phase within 24 hours of incubation. When applied the inhibitors of resveratrol and curcumin that have been approved as Aβ inhibitors in Alzheimer's disease clinical treatment, the latency phase was prolonged 1-2 hours that Aβ enters into accumulation at the incubation time of 5-6 hours. After incubation for 15 hours, Aβenters into the plateau, and the maximum fluorescence intensity was notably decreased to 50-60%, compared with Aβ incubated itself. Under the action of propidium iodide, a little of Aβ aggregation was observed within 24 hours of incubation, where Aβ entered into accumulation at incubation time of 6 hours, but the plateau appeared at the incubation time of 10 hours with only 10% of the maximum fluorescence intensity compared with Aβ incubated itself. The capability of α-Mangostin inhibition on Aβ aggregation was stronger than that of propidium iodide, which caused little Aβ aggregation and thereby no accumulation phase appeared within 24 hours of incubation.

In embodiment 3, ELISA was applied to determine that α-Mangostin decreases Aβ oligomer.

1mg Aβ was dissolved in 500µL HFIP at room temperature, of which 100µL was transferred into a clean centrifuge tube (1.52mL), where 900µL sterile deionized water was added. Followed with centrifugation, the supernatant was moved to another clean centrifuge tube (1.52mL) and HFIP was dried with high pure nitrogen blowing. Then the solution was stirred at 500rpm for 48 hours at 22°C, using a micro stir bar, to get the solution of Aβ oligomer (1µM). In inhibition experiment, α-Mangostin was added into the Aβ oligomer solution to obtain the final concentrations of 5µM, 2µM, 1µM, 0.5µM and 0.2µM, respectively, and followed with another 12 hours stirring at 500rpm and 22°C.α-Mangostin can also be added before stirring with Aβ oligomer for 12 hours.

The incubated solution was applied into the 96-well microplate coated with 100µL monoclonal anti-Aβ antibody (6E10). After incubation for 1 hour at 37°C, the wells were washed 3 times with washing buffer and 100µL Oligomer-specific antibody (A11) was applied. After 5 times washing, 100µL HRP-conjugated Goat anti-Rabbit IgG was applied. After another 5 times washing and 15 minutes colour developing, the optical density was recorded and statistically analyzed with plate reader.

As shown in Fig. 4 and 5, Aβ oligomer concentration was linear with its optical density (OD450) in ELISA, in a the range of 10⁻⁹ to10⁻⁶M, R²=0.98, which indicated it was feasible to determine that α-Mangostin decreases Aβ oligomer with ELISA in that concentration range. α-Mangostin can inhibit formation of Aβ oligomer and disaggregate the formed Aβ oligomer in a concentration dependent manner. When mole ratio of α-Mangostin to Aβ oligomer was 5:1 by applying 5µM α-Mangostin, the formation of Aβ oligomer was inhibited to 14.15±2.86% and the formed Aβ oligomer was disaggregated to 39.58±3.25%. IC₅₀ values of α-Mangostin inhibition and disaggregation on Aβ oligomer were 1.09±0.54µM and 1.59±0.82µM, respectively.

In embodiment 4, neuroprotective effect of α-Mangostin was analyzed with high content assay.

Embryonic day 15 SD rats are etherized and embryos were collected to a sterile dish with an anatomic buffer. Heads of the embryos were cut to a precooling dish with an anatomic buffer. A pair of straight forceps were inserted through eyes to fix the head, and another pincett was inserted along the head sagittal suture. The mening and skull were torn from front to back and to collect the brain. The hippocampus was peeled to the anatomic buffer in a precooling dish. The hippocampus was dissected by scissors disinfected with 75% alcohol. The minced tissues were collected into a marked 15ml centrifuge tube and let them naturally precipitate to the bottom of the tube. The supernatant was discarded, and 2mL 0.05% Trypsin was added. The tube was flipped several times and let it incubate for 5 minutes at 37°C (shake evenly every two minutes). The incubation was stopped, and cells were separated by fine glass pipetting. After standing for 5 minutes, and the connective tissues were collected at the bottom of the tube. The supernatant was centrifuged, and the obtained precipitate was loosed, to which 2mL DMEM buffer with 10% FBS was added. 0.1µL of the mixture was taken to Cell counting. Then the cells were planted on the microplate after dilution and incubated at 37°C and 5% CO₂. The buffer was replaced by 1ml fresh neuron culture medium.

The live cell dye stock in CELLOMICS ® Multiparameter Cytotoxicity 2 Kit was diluted to its working concentration. 50µL/well of the dye solution was added to the 96 well plate, where primary neuronal cells are cultured. After incubation for 30 minutes at 37°C and 5% CO₂, the supernatant is discarded gently and fixation fluid prewarmed at 37°C is added. Then the supernatant was discarded gently and the wells were washed with a washing buffer of 100µL/well. The supernatant was discarded gently again and 100µL/well of nucleus dye was added. After incubation in dark for 10 minutes at room temperature, the wells were washed with washing buffer of 100µL/well. Then 200µL/well washing buffer was added to seal the plate. The fluorescence intensity was measured with high content plate reader and analyzed by the software of Cell Health Profiling Bio Application.

As shown in Fig. 6, 1µM Aβ oligomer had direct neurotoxicity on primary neuronal cells. It destroyed the cell morphology and neuronal functions, which performed as the effects on nuclear size, membrane permeability and mitochondrial membrane potential. α-Mangostin had the neuroprotective effect against Aβ oligomer in a bell shaped and concentration dependent manner. For example, 50nM α-Mangostin restored the cell membrane permeability of neuronal cells from 173.75±6.82% to 107.75±9.39%, and mitochondrial membrane potential from 70.25±6.97% to 105.25±5.84%, which showed significant difference between medicated group and model group (P<0.01) but no difference compared with the normal control (P>0.05).

In embodiment 5, cognitive improvement of α-Mangostin was measured in SAM-P8 mice by a water maze test.

SAM-P8 mice were weight and divided into five groups randomly, including the model group, low-dose (1µg/kg) , mid-dose (10µg/kg) , high-dose (100µg/kg) groups and positive control group treated with resveratrol (10mg/kg). SAM-R1 mice were used as normal control. Reagents were mixed in food and applied to mice from the 3rd day after grouping (enter 6 months age) to the end of the test. Normal control group and model group were administrated with the same amount of food.

Morris water maze test was carried out when SAM-P8 mice entered the age of 8 months. Water temperature in the experiment was controlled at 22±0.5°C. Mice were put into water with their faces towards the wall and backs towards water. The computer started automatically to record the trace and time (escape latency) of mice seeking the platform within 100 seconds. If mice failed to reach the platform within 100 seconds, they were guided to swim straightly to the platform and stand there for 30 seconds. The tests were carried out twice every day, with an interval of 6 hours, and continued for 3 days. After place navigation test, the platform was removed. Mice were placed into water at a certain place of entry. The first time those mice reached the platform and the times that mice across the platform were recorded.

As shown in Fig. 7, with the proceeding of training, although the escape latency and distance of the mice including the model group were all decreased, these two parameters of the model group obviously increased compared with that of the normal control (P<0.05 and P<0.01). α-Mangostin significantly reduced the escape latency and distance. Especially in the experiment of day 2, α-Mangostin performed much more effective in reducing escape latency than that of resveratrol treated as the positive group. The escape latency of mid-dose group, administrated with 10µg/kg α-Mangostin, was reduced to 46.16±5.51s, which was notably less than that of model group, 72.17±10.09s (P<0.05), and had no significant difference from that of normal control.

From day 3, resveratrol also showed good capability in cognitive improvement that the escape latency and distance were notably decreased compared with that of model group. But α-Mangostin was much more effective. The escape latency of mid-dose group, administrated with 10µg/kg α-Mangostin, was reduced to 40.02±4.16s and 19.05±3.27s, which was notably shorter than that of model group, 55.66±5.51s and 39.93±4.12s (P<0.05and P<0.01), and had no difference from that of normal control. The distance of mid-dose group was also reduced to 438.78±46.02cm and 223.15±31.29cm, which was notably shorter than that of model group, 773.06±65.54s and 543.13±56.72s (P<0.01), and had no difference from that of normal control.

At present, resveratrol entered the phase III and IV clinical trial for Alzheimer's disease in USA, of which the mechanism was considered as the inhibition on Aβ aggregation. Meanwhile, SAM-P8 mice were widely accepted as the animal model in PD studies for Alzheimer's disease. Therefore, the effect of α-mangostin on inhibition Aβ aggregation, neuroprotection and cognitive improvement can be considered as the treatment for pathologic process of Alzheimer's disease.

## Claims

1. Application of α-Mangostin in preparation of a medicament for Alzheimer's disease.

2. The application according to claim 1, wherein the medicament is administered orally.

3. The application according to claim 2, wherein the single dose of α-Mangostin is 50ng/ kg-200µg/kg.

4. The application according to claim 3, wherein the single dose of α-Mangostin is 500ng/ kg-50µg/kg.

5. The application according to claim 1, wherein the medicament is administered by injection.

6. The application according to claim 1, wherein the medicament is in tablet, granule, medicinal capsule or powder-injection forms.
